Europäisches Patentamt

European Patent Office

Office européen des brevets

(19)

(11) Publication number: **0 059 277**

**A1**

(12)

# EUROPEAN PATENT APPLICATION

(21) Application number: **81300844.8**

(22) Date of filing: **02.03.81**

(51) Int. Cl.³: **G 01 N 21/82**
**G 01 N 33/86**

(43) Date of publication of application:
**08.09.82 Bulletin 82/36**

(84) Designated Contracting States:
**AT BE CH DE FR GB IT LI LU NL SE**

(71) Applicant: **J. & P. Coats, Limited**
**155 St. Vincent Street**
**Glasgow G2 5PA Scotland(GB)**

(72) Inventor: **Maini, Roshan**
**Stonedyke Watt Road**
**Bridge of Weir Renfrewshire Scotland(GB)**

(74) Representative: **Ferguson, James MacKay et al,**
**Cruikshank & Fairweather 19 Royal Exchange Square**
**Glasgow, G1 3AE(GB)**

(54) Rapid quantative measurement of fibrinogen in blood plasma.

(57) The invention relates to a method of measuring the concentration of fibrinogen in blood plasma.

The concentration of fibrinogen in a test blood plasma is obtained by measuring the turbidities (optical densities) of reference samples of physically similar dimensions of blood plasmas containing known concentrations of fibrinogen, establishing a relationship between concentration of fibrinogen and turbidity in respect of samples of blood plasma of the said physical dimensions and using said relationship to obtain the fibrinogen concentration in a sample of the test plasma having the same physical dimensions as the reference samples from the measured optical density of that test sample using said determined relationship.

Apparatus for performing the method incorporates an optical system (2, 4) by which a test sample and reference samples of blood plasma may be examined for turbidity and calculating and display means (5) arranged to display the concentration of fibrinogen in the test sample in accordance with the determined relationship.

FIG. 3

RAPID QUANTATIVE MEASUREMENT OF

FIBRINOGEN IN BLOOD PLASMA

The present invention relates to a method of providing a rapid quantative measurement of the proportion of fibrinogen in a sample of blood plasma.

Over the past several years there has been an increasing demand by physicians for estimates to be made of the fibrinogen concentration in the blood plasma of their patients in order to understand their conditions in which the haemostatic system is disturbed. The prime step in the blood coagulation process is the proteolysis by thrombin of circulating fibrinogen. This converts the fibrinogen into insoluble fibrin which is the base material of the blood clot. In any analysis of the propensity of a patient's blood to clot a knowledge of this fibrinogen concentration is important.

Examples of situations where a coagulation system can be disturbed are in conditions of disseminated intravascular coagulation, some conditions in later pregnancy, during major surgery and in conditions of thrombosis. Typically, when the patient's condition is changing very quickly the clinician requires accurate details of the patient's coagulation profile in general, and fibrinogen concentration in particular. On many occasions such details are needed quickly, e.g. to assist in the assessment of thrombolytic therapy or in the diagnosis of disseminated intravascular coagulation. Thus the routine laboratory needs a technique which has the attributes of speed, simplicity and reliability. The problem as far as fibrinogen is concerned is that there has previously been no technique which will encompass all three of these attributes simultaneously. Known accurate methods of measuring fibrinogen are slow and technically complex and are thus inconvenient for routine use on a large scale.

Previous techniques based on the principle of collection of clots formed by thrombin or the method of sulphite precipitation are probably the most accurate but they are technically difficult and very time consuming. An attempt has been made to speed up this type of approach by measuring the rate of change of optical density of a plasma sample while it is clotting but this method still takes about half an hour to complete and is also particularly unreliable in the large number of cases where fibrinolysis is taking place and fibrinogen degradation products are present. The fastest technique is a fibrinogen titre method but this is at best only semi-quantative and gives only a crude estimation of the fibrinogen concentration.

Previous chemical techniques such as the use of ammonium or sodium sulphate have the severe defect that they are not specific for fibrinogen. Their use is based on the "salting-out" principle so, because of the presence of other plasma proteins, they are inherently unreliable, especially with abnormal plasma samples where the fibrinogen concentration is low or the gamma globulin proportion is high. The same defect exists in techniques based on a heat precipitation method. Immunological methods are very specific but are the slowest of all and are therefore of no use in the routine laboratory for fibrinogen measurement.

There has been produced a method of estimating fibrinogen in blood plasma in which the weight of precipitate produced on treating a sample of the plasma with sodium sulphite is measured. While this method gives a more reliable result than the conventional method, when the plasma contains fibrinogen degradation products it is still difficult and time consuming to carry out. This method is therefore unsuitable for frequent routine analysis or for rapidly estimating the degree of hypo- or hyper-fibrinogenaemia suffered by a patient, for example, in thrombolytic therapy.

It is an object of the present invention to provide a method of measuring simply, quickly and with sufficient accuracy the concentration of fibrinogen in a sample of blood plasma and apparatus for performing the method. The concentration of fibrinogen is conveniently expressed in grams/litre (g/l).

A method of providing a rapid quantative measurement to an acceptable degree of accuracy of the concentration of fibrinogen in a sample of blood plasma under test according to the invention comprises the steps of forming a substantially homogeneous mixture of the plasma sample previously treated with an anticoagulent and a 1% to saturated solution of a readily water soluble sulphite, the ratio blood plasma:sulphite solution lying in the range from 5:1 to 1:5, then measuring the turbidity (optical density), expressed in chosen units, of the plasma sample under test providing at least two reference samples of blood plasma which are identical in physical form with the test sample and containing different but known proportions of fibrinogen, measuring and expressing in said chosen units the turbidities of said reference samples so that a particular relationship between fibrinogen concentration and turbidity is determined for these reference samples and then using the measured turbidity of the test sample to obtain the concentration of fibrinogen present in the test sample according to the said particular relationship.

The units used for expressing the turbidity are unimportant since it is the numerical value of the ratio of the turbidity of the test sample and of the reference sample which is the factor required.

Blood plasmas containing known concentrations of fibrinogen are readily available from commercial sources and a commercial blood plasma may be used for the reference plasma. A reference plasma may, however, be made by choosing a conveniently available sample of blood plasma and measuring its fibrinogen concentration accurately using one of the known accurate but slow techniques. Since the turbidity or optical density of a plasma has been found by the present inventors to bear a linear relationship to the proportion of fibrinogen present in it the proportion of fibrinogen present in the reference plasma does not matter provided it is accurately known.

For rapid measurement of the fibrinogen content of a number of plasma samples a graph may be obtained by plotting the numerical values of the concentration of fibrinogen found to be present or known to be present in several plasmas chosen as reference plasmas against the figures obtained for the turbidities of the plasmas, the fibrinogen proportion of each plasma sample under test being obtained directly by reading off the value of fibrinogen proportion on the graph opposite the value of the turbidity of that sample under test. The graph obtained is a straight line.

The blood plasma may be centrifuged with the anti-coagulent before being mixed with the sulphite solution.

The anti-coagulent may be a citrate such as sodium citrate or may be heparin.

The plasma sample to be tested may be either composed of anticoagulated plasma alone or in aqueous solution. Where the plasma is cloudy, for example lipaemic, the sample may be diluted so that the cloudiness is reduced to an extent such that although it will affect the turbidity reading the amount by which the turbidity reading is affected will have a negligible effect on the value obtained for the proportion of fibrinogen present.

Common readily soluble sulphites suitable for use in the method are potassium, sodium, ammonium and magnesium sulphites.

The plasma sample to be tested may be mixed with water, the sulphite being then dissolved in the mixture, or preferably, the sulphite is first dissolved in water and then mixed with plasma which is optionally diluted with water. The latter method is particularly preferred because an aqueous solution of the sulphite can be made up accurately in advance and mixed with the plasma sample in accurate doses. If desired, this accurate dosing can be effected using a preformed container containing a predetermined amount of the sulphite solution. This mixing can be carried out automatically if desired. The water used in the method according to the invention is preferably substantially pure, for example deionized or distilled water.

The amount of the sulphite aqueous solution is preferably double that of the mixture containing plasma and water. Aqueous solution of the sulphite preferably contains 8% to a saturated solution, for example, about 10% (w/v) of the sulphite.

To ensure the formation of a homogeneous mixture of plasma and sulphite the mixture may be allowed to stand for a period which is variable but preferably 30 seconds to 3 minutes.

Apparatus for performing the method of the invention comprises an optical system incorporating a light source and a photoelectric cell, means for holding a sample of blood plasma in a position optically between the light source and the photoelectrical cell, display means arranged to receive the signal from the photoelectric cell and display the reading in chosen units of turbidity and a zeroising control operative to set the display means to indicate zero when a signal representing a particular turbidity is received from the photoelectric cell.

The optical system may be a double beam system and there may be two photoelectric cells, two sets of means for holding plasma samples optically between the light source and a respective photoelectric cell and a switching device operable to bring into use a chosen beam system and the associated photoelectric cell.

The apparatus may incorporate a stirring means for stirring the plasma mixture and may incorporate timing means capable of regulating the time of stirring and/or the time when the mixture is standing after mixing and before the turbidity is measured. The timing means may alternatively or in addition be arranged to stop the means for measuring the turbidity at a chosen interval of time after stirring of the plasma mixture has taken place.

The double beam optical system where used may be so arranged that the beam is directed first through one sample and then through the other, the beam after traversing each sample reaching the same photoelectric cell. This eliminates possible errors by different responses of two different photoelectric cells.

In the accompanying drawings Fig. 1 is a graph showing readings of fibrinogen content obtained by the method of the invention in comparison with readings obtained by a known very accurate method but one which is slow in operation, Fig. 2 illustrates a calibration graph for the reference plasma showing fibrinogen content plotted against optical density and Fig. 3 shows diagrammatically one form of apparatus for use in performing the invention.

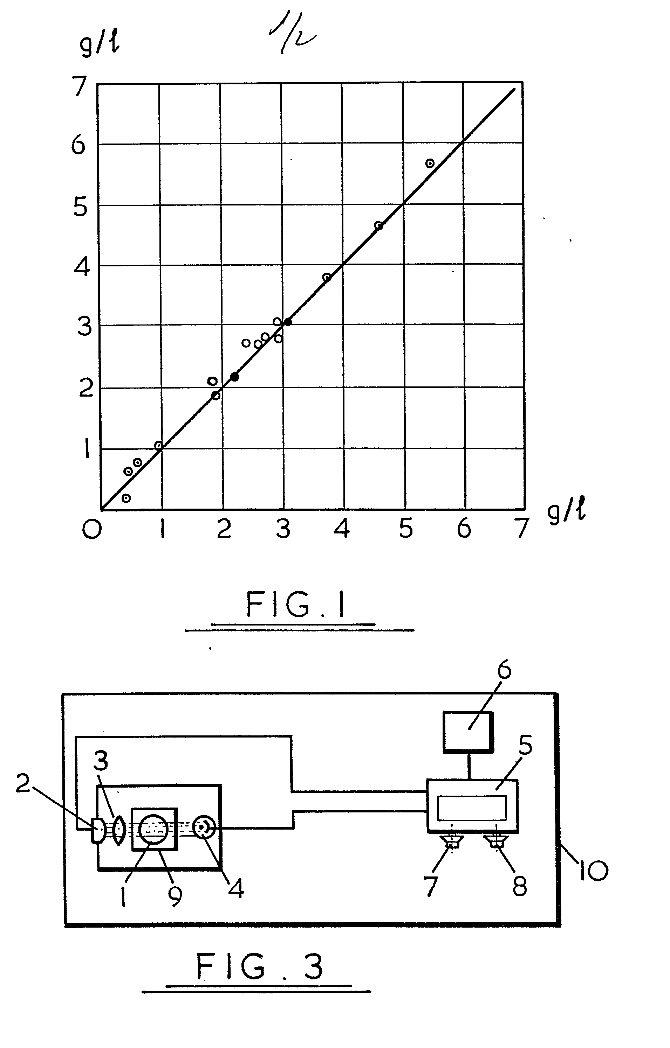

In the drawings and referring first to Fig. 1 the horizontal base line represents concentration of fibrinogen represented in g/l and the vertical base line represents proportions of fibrinogen content obtained by the method of the invention. It will be seen that the graph line is a straight line beginning at the origin of the graph and lying at a slope of 1:1. There is almost complete agreement between the figures obtained by the method of the invention and the figures obtained by the known accurate method of determining fibrinogen content.

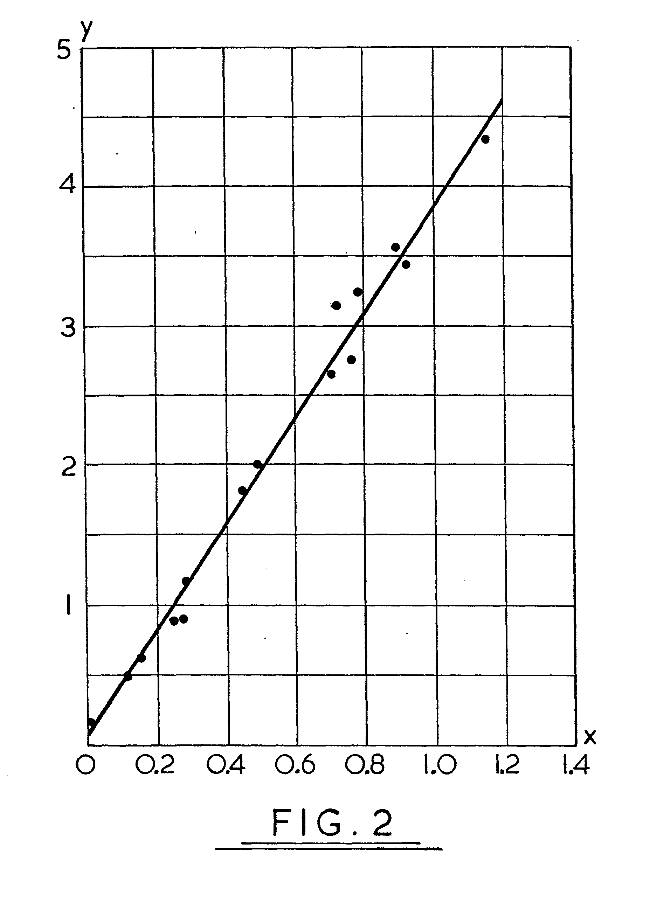

Referring to Fig. 2 the x-axis represents optical densities and the y-axis represents corresponding values of fibrinogen concentration. The optical density may be indicated in any convenient units, for example attenuation of light intensity.  The optical units used do not matter provided they are the same as the units used in obtaining the calibration graph because they are used only for locating the point on the graph for reading off the fibrinogen content of the plasma sample under test.  The fibrinogen concentration as indicated by the ordinate is seen to vary linearly as the turbidity or optical density since the graph is a straight line.

In Fig. 3, 1 denotes a holder for a receptacle e.g. a cuvette or a test tube containing a sample of blood plasma, 2 denotes a source of light, 3 denotes a collimating device, e.g. a condensing lens for directing a beam of light through the container 2.  4 denotes a photoelectric cell arranged to receive the beam of light from the light source 2 after it has passed through the receptacle 1. 5 denotes a display and computing device calibrated in grams/litre (g/l) and programmed to provide a signal bearing a relationship to the intensity of the beam of light falling on the photoelectric cell in accordance with the formula of the graph of Fig. 2.  6 denotes a timer operatively coupled to the display device 5 to cause the display device to become operative at the start of a predetermined interval after a starting switch 7 controlling the timer 6 is moved to the operating position to hold the display on the display device at the instantaneous value indicated after that interval of time.  8 denotes a zeroising switch operative to cause the display device to show a zero reading while said signal is being impressed on it with the object that if a different signal is now applied to the display device it will indicate a value which is the difference between the present signal and the previous signal.  9 denotes a magnetic stirring device for a cuvette or a test tube in the holder 1.  10 denotes a casing enclosing all the operative parts of the apparatus.

In performance of the process a reference sample of blood plasma provides the basis for the calibration graph which is an essential part of the process. This
reference plasma may be obtained from one of the producers
specializing in the supply of such samples. To obtain
the graph such as is illustrated in Fig. 2 it is necessary
to make a number of samples having different concentrations
of fibrinogen. The easiest method of doing this is to
obtain quantities of plasmas having known concentrations
of fibrinogen. Alternatively one plasma may be used
as a basis to make a number of samples having different
concentrations of fibrinogen. One method of doing this
is by centrifuging the plasma which had been previously
treated with an anticoagulant with a 0.15 molar saline
solution or by mixing it with quantities of concentrated
fibrinogen solution obtained by extracting the serum from
other blood samples. The different samples having
different concentrations of fibrinogen can then be
examined for fibrinogen content by one of the known
accurate but slow methods. The length of time it takes to
process these samples is not important because calibration
is normally a single operation which requires to be
performed once only.

The figures obtained from the different samples
containing different fibrinogen proportion are then
plotted to form a graph in which the x axis is divided
into units of optical density and the y axis units of
fibrinogen concentration. With reference to the graph
of Fig. 2 which was obtained by actual experiment the
formula of this graph giving the relationship between
optical density and fibrinogen concentration is as
follows:-

$$\phi = 3.83A + 0.05$$

where $\phi$ is fibrinogen concentration in g/l and A is the
optical density in any chosen units.

The apparatus illustrated in diagrammatic form
in Fig. 3 is programmed so that when a signal representing
any particular value A is applied to it the indication on
the display is the value of $\phi$ as given by the formula for the relevant graph for that applied value of A.

The programming of the display device remains constant and where the device of Fig. 3 is to be used it is not necessary once the display device has been programmed to refer further to the actual graph, although it is desirable that at intervals the apparatus should be calibrated against a blood plasma sample of known fibrinogen content to ensure that it is still operating as originally calibrated.

Using the apparatus of Fig. 3 the sample of blood plasma of which the fibrinogen content is to be obtained is divided into two equal portions one of which is to be used as a reference sample and the other which will be the test sample and the two portions put into identical cuvettes or test test tubes or other containers which make it possible to pass light through the samples.

Each portion may contain for example 0.5 ml of plasma. Into the cuvette containing the reference sample 1 ml of say 0.9% saline solution is added. This mixture forms the base from which the optical measurement of the test sample in the other cuvette will be made.

The cuvette containing the saline solution and plasma is shaken enough to ensure good mixing and is then placed into the optical measuring section of the apparatus. When the sample is placed in the measuring section the digital display indicates a random reading. The display is then zeroised to form the base referred to above.

The blood plasma sample to be tested is placed in the holder in the apparatus. A quantity of dissolved anhydrous sodium sulphite is then added to the sample in the sample holder. Where the sample contains 0.5 ml of plasma the quantity of sodium sulphite may be 1 ml. The stirring device 9 and the timer 6 are then switched on by operating the starting switch 7 and the mixture is stirred until it is substantially homogeneous. It is then left undisturbed to allow the sodium sulphite to bond with the fibrinogen molecules so that the fibrinogen becomes precipitated out of the solution. After 30 seconds the timer 6 operates to hold the display on the display device constant. The figure now displayed on the display device has been subtracted from the original figure obtained from the reference sample and shows the actual fibrinogen content as a direct reading on the display. These may be shown in practical units such as g/l.

The method may be performed using a simple spectrophotometer or other optical measuring device to provide the comparative readings of turbidity or optical density and the graph to provide the corresponding figures of fibrinogen content but the apparatus described makes performance of the method easier and faster and reduces considerably the possibilities for mistakes.

0059277

## CLAIMS

1. A method of providing a rapid quantative measurement to an acceptable degree of accuracy of the concentration of fibrinogen in a sample of blood plasma under test characterized by forming a substantially homogeneous mixture of the plasma sample previously treated with an anticoagulant and a 1% to saturated solution of a readily water soluble sulphite, the ratio blood plasma:sulphite solution lying in the range from 5:1 to 1:5, then measuring the turbidity (optical density), expressed in chosen units, of the plasma sample under test, providing at least two reference samples of blood plasma which are identical in physical form with the test sample and containing different but known proportions of fibrinogen, measuring and expressing in said chosen units the turbidities of said reference samples so that a particular relationship between fibrinogen concentration and turbidity is determined for these reference samples and then using the measured turbidity of the test sample to obtain the concentration of fibrinogen present in the test sample according to the said particular relationship.

2. The method according to claim 1 characterized by preparing a graph obtained by plotting the numerical values of the concentration of fibrinogen found to be present or known to be present in several plasmas chosen as reference plasmas against the figures obtained for the turbidities of the plasmas and obtaining the measurement of the fibrinogen concentration of a plasma sample under test by reading off the concentration of fibrinogen on the graph opposite the figure of the turbidity of the sample under test.

3. The method according to claim 1 characterized in that the sample of blood plasma under test contains plasma in aqueous solution.

4. The method according to claim 1 characterized in that the plasma of the sample under test is diluted so that any cloudiness present is reduced to an extent such that although it will still affect the turbidity reading the amount by which the turbidity reading is affected will have a negligible effect on the figure obtained for the proportion of fibrinogen present.

5. Apparatus for performing the method claimed in claim 1 characterized by incorporating an optical system incorporating a light source (2) and a photoelectric cell (4), means (1) for holding a sample of blood plasma in a position optically between the light source and the photoelectric cell, display means (5) arranged to receive the signal from the photoelectric cell and display the reading in chosen units and a zeroising device (8) operative to set the display means to indicate zero while a signal representing a particular turbidity is being received from the photoelectric cell.

6. Apparatus according to claim 5, characterized in that a memory is included in the apparatus, that the zeroising device (8) is operative when activated to transfer the reading from the display to the memory as part of the operation of zeroising the display and that there is included means operative to subtract algebraically the information contained in the memory from the figure corresponding with the next signal received from the photoelectric cell and display the difference figure on the display.

7. Apparatus according to claim 5 characterized by incorporating a stirring means (9) for stirring the plasma mixture.

8. Apparatus according to claim 5 characterized by incorporating timing means (6) capable of regulating the intervals of time between different operations performed by the apparatus.

g/l          1/2

## FIG.1

## FIG.3

FIG. 2

European Patent Office

**EUROPEAN SEARCH REPORT**

Application number

EP 81 30 0844

## DOCUMENTS CONSIDERED TO BE RELEVANT

| Category | Citation of document with indication, where appropriate, of relevant passages | Relevant to claim |
|---|---|---|
| A | CHEMICAL ABSTRACTS., vol. 84, 1976, page 216, abstract 132271h Columbus, Ohio, USA M.W. RAMPLING et al. "The sulfite precipitation method for fibrinogen measurement; its use on small samples in the presence of fibrinogen degradation products" & CLIN. CHIM. ACTA 1976, 67(1), 43-52. <br> * Whole abstract * | 1 |
| A | CHEMICAL ABSTRACTS, vol. 63, 1965, column 18633f Columbus, Ohio, US D.W. HUNTER et al. "Modified fibrinogen assay" & TECH. BULL. REGISTRY MED. TECHNOLOGISTS, 35(8), 145-9 (1965) <br> * Whole abstract * | 1 |
| A | CHEMICAL ABSTRACTS, vol. 68, 1968, page 2645, abstract 27454s Columbus, Ohio, US J.F. GOODWIN et al. "An evaluation of turbidimetric techniques for estimation of plasma fibrinogen" & CLIN. CHEM. 13(12), 1057-64 (1967) <br> * Whole abstract * | 1 |
| A | US - A - 4 043 676 (O. HOLZINGER et al.) <br> * Columns 2,3; figures 1,2 * | 5 |

./.

### CLASSIFICATION OF THE APPLICATION (Int Cl.)

G 01 N 21/82
33/86

### TECHNICAL FIELDS SEARCHED (Int Cl.)

G 01 N 33/86
33/48
21/82

### CATEGORY OF CITED DOCUMENTS

X: particularly relevant
A technological background
O. non-written disclosure
P: intermediate document
T. theory or principle underlying the invention
E conflicting application
D: document cited in the application
L citation for other reasons

& member of the same patent family.
corresponding document

The present search report has been drawn up for all claims

| Place of search | Date of completion of the search | Examiner |
|---|---|---|
| The Hague | 02-11-1981 | BOEHM |

EPO Form 1503.1 06.78

European Patent
Office

**EUROPEAN SEARCH REPORT**

| | DOCUMENTS CONSIDERED TO BE RELEVANT | | CLASSIFICATION OF THE APPLICATION (Int. Cl.3) |
|---|---|---|---|
| Category | Citation of document with indication, where appropriate, of relevant passages | Relevant to claim | |
| A | FR - A - 2 448 145 (L'OREAL)<br>* Pages 2-3,9-10; figure 1 * | 6 | |
| A | US - A - 3 932 040 (H. WARNCKE)<br>* Columns 4,5; figure 1 * | 6 | |
| A | E. SANDELL: "COLORIMETRIC DETER-<br>MINATION OF TRACES OF METALS", 2nd<br>edition- Ed. Interscience, 1950<br>New York, US<br>Chapter III: Colorimetry and spec-<br>trophotometry in trace analysis<br>"Standard Curves"<br>pages 71-72<br><br>* Whole document * | 2 | TECHNICAL FIELDS SEARCHED (Int. Cl.3) |
| A | F. WELCHER "STANDARD METHODS OF<br>CHEMICAL ANALYSIS", 6th edition -<br>Ed. Van Nostrand, 1966,vol.II,Part A<br>Princeton, US<br>Chapter 30: Chemical analysis<br>in clinical medicine "Fibrinogen"<br>page 1093<br><br>* Whole document * | 1,2 | |
| A | EP - A - 0 010 526 (SALUS<br>I.D.C. Dr. FRANCESCO)<br>* Front page, abstract * | 7,8 | |

EPO Form 1503.2   06.78